# FASCICULE DE BREVET EUROPEEN

(11) **EP 2 803 400 B1**
(45) Date de publication et mention de la délivrance du brevet: **16.03.2016**
(21) Numéro de dépôt: 14168408.4
(22) Date de dépôt: 15.05.2014
(51) Int. Cl.: B01D 53/00, B01D 53/32, B01J 19/08, B01J 19/12, B01J 19/24

(54) **DISPOSITIF DE TRAITEMENT DE GAZ ET SA MÉTHODE D'ASSEMBLAGE, SYSTÈME ET PROCÉDÉ DE TRAITEMENT DE GAZ**
VORRICHTUNG ZUR GASAUFBEREITUNG UND ENTSPRECHENDE METHODE ZUM ZUSAMMENBAU SOWIE SYSTEM UND VERFAHREN ZUR GASAUFBEREITUNG
GAS TREATMENT DEVICE AND METHOD FOR ASSEMBLING SAME, GAS TREATMENT SYSTEM AND METHOD

(30) Priorité: 15.05.2013 FR 1354353
(43) Date de publication de la demande: 19.11.2014
(73) Titulaire: Compagnie Industrielle D'Applications Thermiques, 01350 Culoz (FR); Ecole Nationale Supérieure de Chimie de Rennes, 35708 Rennes Cedex 7 (FR); Centre National de la Recherche Scientifique (CNRS), 75016 Paris (FR)
(72) Inventeur: Petit, Philippe, 73310 Ruffieux (FR); Vialle, Pierre-Jean, 38780 Estrablin (FR); Maciuca, Alina, 86000 Poitiers (FR); Batiot-Dupeyrat, Catherine, 86800 Sevres Anxaumont (FR); Tati-Bouet, Jean-Michel, 86000 Poitiers (FR); Assadi, Aymen Amin, 6080 Gabes (TN); Bouzaza, Abdelkrim, 35510 Cesson-Sevigne (FR); Wolbert, Dominique, 35510 Cesson-Sevigne (FR)
(74) Mandataire: Myon, Gérard Jean-Pierre

(56) Documents cités:
- WO-A1-2007/051912
- US-A1- 2008 170 971
- SANO T ET AL: "Contributions of photocatalytic/catalytic activities of TiO2 and gamma-Al2O3 in nonthermal plasma on oxidation of acetaldehyde and CO", JOURNAL OF MOLECULAR CATALYSIS A: CHEMICAL, ELSEVIER, AMSTERDAM, NL, vol. 245, no. 1-2, 15 février 2006 (2006-02-15), pages 235-241, XP028015539, ISSN: 1381-1169, DOI: 10.1016/J.MOLCATA.2005.10.002 [extrait le 2006-02-15]

## Description

La présente invention concerne un dispositif de traitement de gaz, une méthode d'assemblage de ce dispositif, ainsi qu'un système et un procédé de traitement de gaz.

La photocatalyse permet d'initier une réaction chimique se produisant à la surface d'une substance nommée « photocatalyseur », lorsque cette dernière absorbe des photons. Généralement, le photocatalyseur est constitué de dioxyde de titane.

La photocatalyse est utilisée pour éliminer les polluants organiques présents dans l'air. Une lampe émet un rayonnement photonique, généralement ultraviolet, éclairant le photocatalyseur, qui devient alors un puissant oxydant détruisant les composés organiques volatils (COV). Plus précisément, lorsqu'il est soumis à un rayonnement photonique, le photocatalyseur génère des espèces oxygénées très réactives à sa surface, qui dégradent les composés organiques jusqu'à ce que le carbone des chaînes carbonées soit complètement transformé en dioxyde de carbone.

WO-2009/007588 divulgue une unité de traitement de gaz par photocatalyse, comprenant un support diélectrique plat dont les deux faces comportent chacune une bande conductrice formant une électrode. Un générateur électrique alimente les électrodes, qui génèrent un plasma de surface contre le photocatalyseur. En fonctionnement, le plasma de surface constitue une source stable du rayonnement qui active le catalyseur. De cette manière, la fonction du plasma est la même que celle du rayonnement UV de la lampe. Eventuellement, plusieurs unités de traitement de gaz sont disposées parallèlement dans un châssis comportant une entrée et une sortie pour le gaz, formant plusieurs canaux de circulation. Toutefois, le rayonnement UV du plasma est inférieur à celui généré par une lampe, de sorte que cette unité de traitement a un faible rendement qui ne lui permet pas d'être utilisé pour des applications à l'échelle industrielle.

WO-2007/051912 et US-2008/170971 divulguent un dispositif de traitement d'effluents gazeux dans lequel le photocatalyseur est activé à la fois par des lampes, réparties à l'extérieur du dispositif et émettant un rayonnement ultraviolet, et par un plasma froid volumique généré par des électrodes alimentées par un générateur électrique. L'efficacité de ces dispositifs de traitement est meilleure que l'unité de traitement de WO-2009/007588, dans laquelle le catalyseur est activé uniquement par le rayonnement UV généré par un plasma froid.

Par ailleurs, dans WO-2007/051912, le photocatalyseur peut être placé perpendiculairement aux électrodes et au flux d'air, et se présente alors sous la forme d'un support perméable, par exemple en nid d'abeilles. En alternative, le photocatalyseur est disposé parallèlement aux électrodes et aux flux d'air, et se présente alors sous la forme d'une succession de couches parallèles. La fabrication d'un tel dispositif est relativement longue et coûteuse, compte tenu de l'agencement du photocatalyseur. De plus, le rayonnement ultraviolet des lampes ne permet pas une irradiation optimale du photocatalyseur car les lampes sont localisées à l'extérieur du dispositif et ont une action limitée car elles n'éclairent que localement le photocatalyseur. Par conséquent, un tel dispositif n'est pas adapté à des applications industrielles.

C'est à ces inconvénients qu'entend plus particulièrement remédier l'invention en proposant un dispositif de traitement de gaz associant le traitement par photocatalyse et par plasma, combinant les effets des rayonnements ultraviolets sur le photocatalyseur et d'un plasma froid de surface. L'invention propose un dispositif efficace pour réduire rapidement la concentration des polluants, facile et rapide à fabriquer et ayant un coût de fabrication réduit.

A cet effet, l'invention a pour objet un dispositif de traitement de gaz, comprenant un caisson divisé en plusieurs canaux comportant chacun une entrée et une sortie de gaz. Au moins une lampe générant un rayonnement photonique, notamment ultraviolet, étant placée dans chaque canal. Chaque canal étant délimité par deux parois de cloisonnement comprenant chacune :
- une paroi diélectrique,
- un élément photocatalytique disposé contre la paroi diélectrique du côté du canal et comprenant un support qui supporte un photocatalyseur,
- une première électrode disposée contre l'élément photocatalytique, et
- une deuxième électrode disposée contre la paroi diélectrique.

Grâce à l'invention, le traitement du gaz est efficace, compte tenu notamment des lampes qui sont placées à l'intérieur de chaque canal, ce qui permet à une majorité du rayonnement photonique d'atteindre le photocatalyseur. De plus, la présence de plusieurs canaux confère au dispositif un rendement satisfaisant permettant son utilisation à l'échelle industrielle. Par ailleurs, la fabrication du dispositif de traitement de gaz est aisée, par empilement successif, à l'intérieur du caisson, des éléments de chaque canal.

Selon des aspects avantageux mais non obligatoires de l'invention, un tel dispositif de traitement de gaz peut incorporer une ou plusieurs des caractéristiques suivantes, prises dans toute combinaison techniquement admissible :
- Chaque canal présente une section transversale allongée et, le long de chaque canal, sont disposées plusieurs lampes comprenant chacune un tube fluorescent qui s'étend notamment parallèlement à la plus grande dimension de la section transversale du canal.
- Chaque élément photocatalytique se présente sous la forme d'une feuille et/ou chaque première électrode se présente sous la forme d'une grille.
- Chaque seconde électrode se présente sous la forme d'une plaque.
- Chaque électrode comprend des moyens de raccordement électrique, les moyens de raccordement électrique de chaque première électrode sont situés au niveau d'un premier de ses bords, et les moyens de raccordement électrique de chaque deuxième électrode sont situés au niveau d'un second de ses bords, opposé audit premier bord des premières électrodes.
- Les bords de chaque deuxième électrode sont en retrait vers l'intérieur du canal par rapport aux bords des parois diélectriques.

L'invention concerne également une méthode d'assemblage d'un tel dispositif, la méthode comprenant des étapes dans lesquelles :
- des parois du caisson sont assemblées entre elles de manière à former un ensemble présentant une section transversale en U,
- les éléments délimitant chaque canal, dont les premières électrodes et les deuxièmes électrodes sont disposés successivement contre le fond de cet ensemble, des entretoises définissant la largeur de chaque canal étant intercalées entre les parois de cloisonnement,
- une paroi supplémentaire du caisson est assemblée de manière à ce que la section transversale du caisson présente un contour fermé,
- les lampes sont insérées dans des lumières ménagées dans l'une des parois du caisson et fixées en place dans les canaux.

L'invention concerne également un système de traitement de gaz, comprenant :
- un tel dispositif,
- un premier générateur électrique alternatif alimentant les lampes,
- un deuxième générateur électrique alternatif alimentant les électrodes,
- un organe de mise en dépression faisant circuler le gaz à traiter entre l'entrée et la sortie de chaque canal.

L'invention concerne également un procédé de traitement de gaz au moyen d'un tel système, comprenant des étapes dans lesquelles :
- l'organe de mise en dépression fait circuler le gaz entre l'entrée et la sortie de chaque canal,
- les électrodes génèrent un plasma froid de surface contre les éléments photocatalytiques,
- les lampes génèrent un rayonnement photonique vers les éléments photocatalytiques.

Avantageusement, les étapes sont simultanées.

L'invention sera mieux comprise et d'autres avantages de celle-ci apparaitront plus clairement à la lumière de la description qui va suivre d'un dispositif de traitement de gaz et de sa méthode d'assemblage, ainsi que d'un système et d'un procédé de traitement de gaz conformes à l'invention, donnée uniquement à titre d'exemple et faite en référence aux dessins annexés dans lesquels :
- la figure 1 est une vue en perspective d'un dispositif de traitement de gaz conforme à l'invention ;
- la figure 2 est une vue à plus grande échelle du détail II à la figure 1, un cadre de jonction n'étant pas représenté ;
- la figure 3 est une coupe transversale du dispositif de la figure 1 ;
- la figure 4 est une vue à plus grande échelle du détail IV à la figure 3 ; et
- la figure 5 est une coupe longitudinale du dispositif de la figure 1.

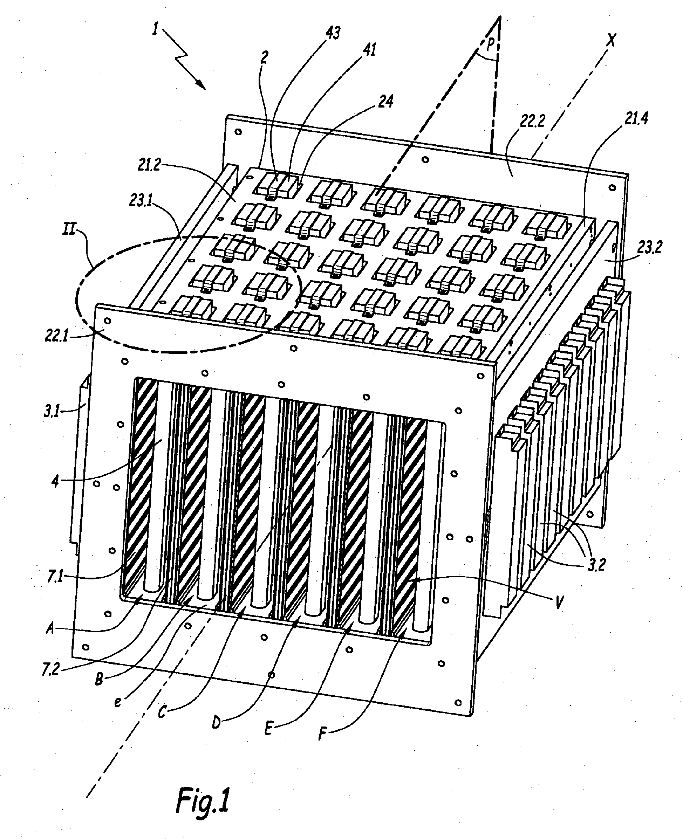

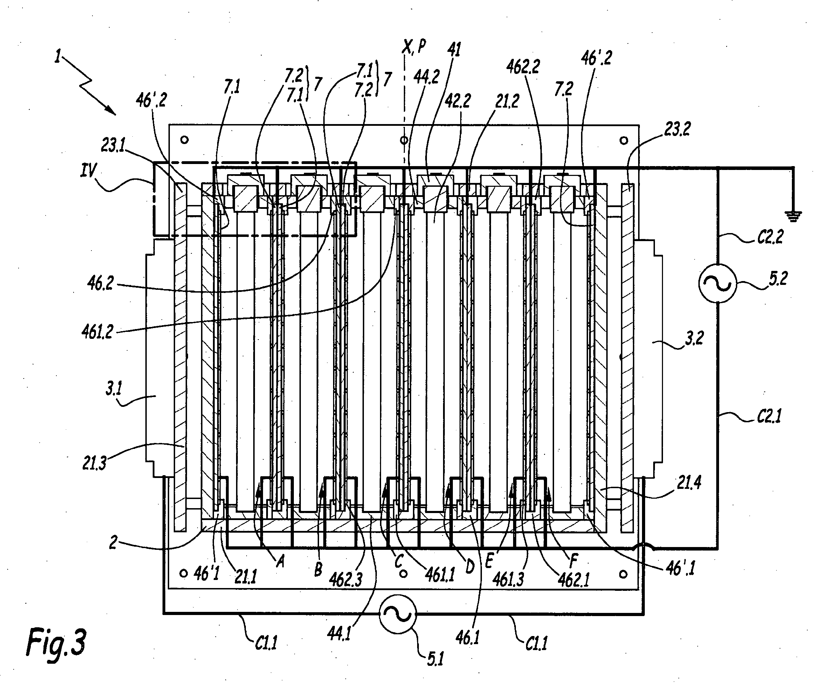

Les figures 1 à 5 montrent un système de traitement de gaz, comprenant un dispositif de traitement de gaz 1 raccordé à deux générateurs électriques alternatifs 5.1 et 5.2, représentés à la figure 3 uniquement. Le dispositif 1 comprend un caisson 2 de forme allongée, creux et parallélépipédique, délimitant un volume intérieur V et s'étendant le long d'un axe géométrique longitudinal X. Le dispositif 1 est globalement symétrique par rapport à un plan médian longitudinal P.

Dans la présente demande, l'adjectif « longitudinal » fait référence à la direction de l'axe X, à l'inverse de l'adjectif « transversal » qui désigne les éléments perpendiculaires à cet axe. De plus, l'adjectif « supérieur » désigne les éléments situés en partie haute des figures 1 à 4, à l'inverse de l'adjectif « inférieur » qui fait référence aux éléments situés en partie basse de ces figures, étant entendu qu'en service, l'orientation du dispositif 1 dans l'espace peut être différente. Toutefois, il est préférable d'utiliser l'orientation correspondant à celles des figures.

Le caisson 2 présente une section transversale globalement carrée et il comprend quatre parois longitudinales et jointives, globalement rectangulaires, à savoir une paroi inférieure 21.1 et une paroi supérieure 21.2 parallèles entre elles, ainsi que deux parois latérales 21.3 et 21.4 perpendiculaires aux parois 21.1 et 21.2.

Le volume intérieur V du caisson 2 est compartimenté, autrement dit divisé en six canaux A, B, C, D, E et F longitudinaux. Les extrémités longitudinales de chaque canal A à F sont ouvertes et forment respectivement une entrée e et une sortie s de gaz. Les canaux A à F présentent chacun une section transversale allongée, dont la longueur et la largeur sont orientées respectivement parallèlement et perpendiculairement au plan P.

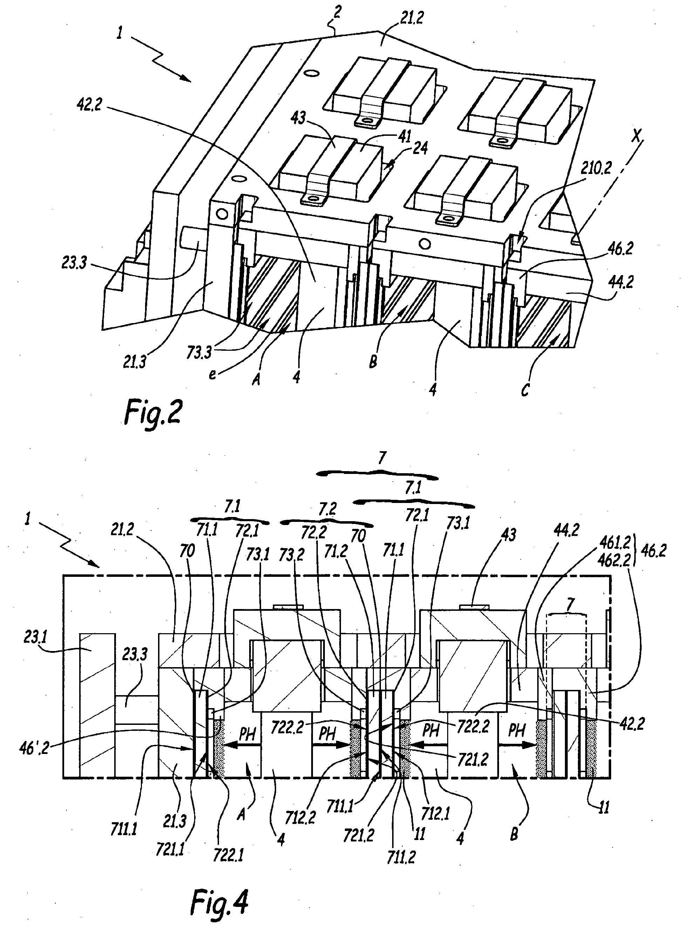

Un cadre de jonction 22.1 ou 22.2 transversal est fixé sur chaque extrémité longitudinale du caisson 2 et comporte une ouverture dont les dimensions correspondent à celles du volume intérieur V du caisson 2, de sorte que les cadres de jonction 22.1 et 22.2 n'obstruent pas l'entrée e et la sortie s des canaux A à F. Le cadre de jonction 22.1 situé au premier plan à la figure 1 n'est pas représenté à la figure 2 pour laisser apparaitre l'entrée e des canaux A, B et C. Les cadres de jonction 22.1 et 22.2 sont utilisés pour raccorder plusieurs dispositifs 1 les uns aux autres ou pour raccorder le dispositif 1 à un caisson d'amenée ou d'évacuation de gaz.

Les canaux A à F sont délimités chacun de part et d'autre par une première paroi de cloisonnement 7.1, située du côté gauche de chaque canal A à F sur les figures 1 à 5, et par une deuxième paroi de cloisonnement 7.2, située du côté droit de chaque canal A à F sur ces figures. Les parois de cloisonnement 7.1 et 7.2 sont parallèles au plan P.

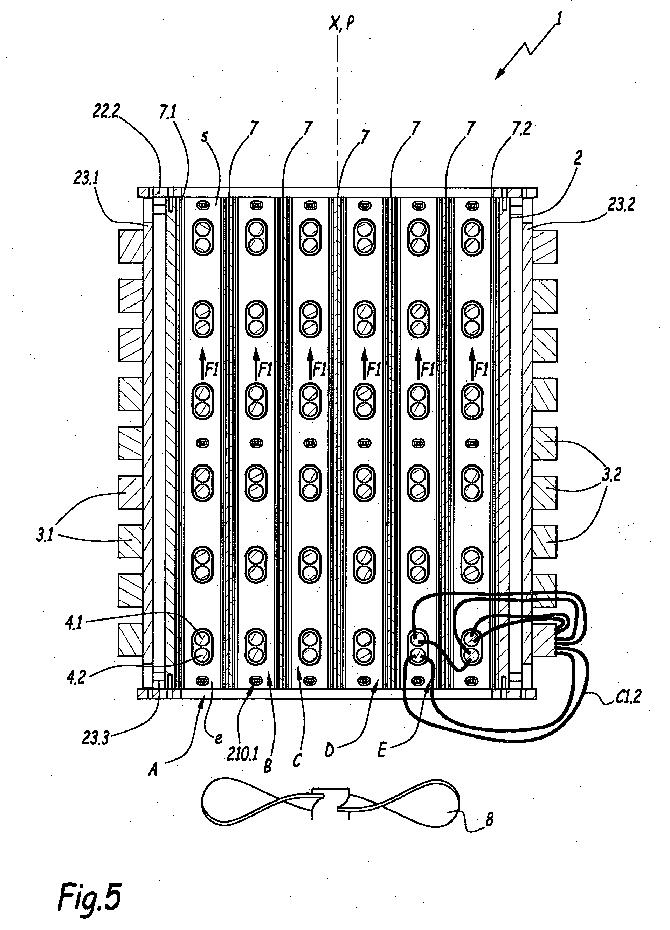

Un organe de mise en dépression 8, tel qu'un ventilateur représenté schématiquement à la figure 5 uniquement, fait circuler le gaz à traiter dans le volume V du caisson 2 parallèlement à l'axe X, entre l'entrée e et la sortie s de chaque canal A à F, comme représenté par les flèches F1.

Comme visible à la figure 4, chaque paroi de cloisonnement 7.1 et 7.2 comprend respectivement une plaque 71.1 ou 71.2 réalisée à partir d'un matériau diélectrique tel que le verre, de dimensions identiques, comportant chacune respectivement une face arrière 711.1 ou 711.2 tournée à l'opposé du canal A, B, C, D, E ou F délimité par cette plaque diélectrique 71.1 ou 71.2, ainsi qu'une face avant 712.1 ou 712.2, opposée et parallèle à la face arrière 711.1 ou 711.2.

Un élément photocatalytique 72.1 ou 72.2 est disposé contre la face avant 712.1 ou 712.2 de chaque plaque diélectrique 71.1 et 71.2, du côté du canal A à F. Les éléments photocatalytiques 72.1 ou 72.2 se présentent chacun sous la forme d'une feuille comportant une face arrière 721.1 ou 721.2 en appui respectivement contre la face avant 712.1 ou 712.2 de la plaque diélectrique 71.1 ou 71.2, ainsi qu'une face avant 722.1 ou 722.2 tournée vers le volume du canal A, B, C, D, E ou F délimité par la paroi de cloisonnement 7.1 ou 7.2 correspondante. Chaque élément photocatalytique 72.1 et 72.2 comprend un support supportant un photocatalyseur déposé sur sa face avant 722.1 ou 722.2. Par exemple, le support est en fibres de verre et le photocatalyseur est du dioxyde de titane TiO₂.

Une première électrode 73.1 ou 73.2 est plaquée respectivement contre la face avant 722.1 ou 722.2 de l'élément photocatalytique 72.1 ou 72.2. Chaque première électrode 73.1 et 73.2 se présente sous la forme d'une grille plate rectangulaire, réalisée à partir d'une plaque métallique en acier inoxydable dans laquelle sont usinées des lumières 73.3 rectangulaires. De cette manière, au niveau de chaque lumière 73.3, la face avant 722.1 ou 722.2 de chaque élément photocatalytique 72.1 et 72.2 n'est pas recouverte par les branches métalliques qui forment la grille et débouche ainsi dans le volume intérieur de l'un des canaux A à F. Certaines branches de la grille peuvent avoir une section plus importante, de manière à former des montants qui améliorent la tenue mécanique de la grille.

Une deuxième électrode 70 se présentant sous la forme d'une feuille rectangulaire pleine réalisée à partir d'un matériau conducteur, par exemple un matériau métallique tel que le cuivre, est disposée contre la face arrière 711.1 ou 711.2 de chaque paroi diélectrique 71.1 et 71.2, à l'opposé du canal A à F délimité par cette paroi. Pour la première paroi de cloisonnement 7.1 du canal A, qui est situé à gauche sur les figures 1 à 5, la deuxième électrode 70 est intercalée entre la paroi latérale 21.3 du caisson 2 et la plaque diélectrique 71.1. De même, pour la deuxième paroi de cloisonnement 7.2 du canal F, qui est situé à droite sur les figures 1, 3, 4 et 5, la deuxième électrode 70 est intercalée entre la paroi latérale 21.4 du caisson 2 et la plaque diélectrique 71.2.

Les ensembles de deux canaux A à F adjacents, c'est-à-dire les canaux A et B, B et C, C et D, D et E, ainsi que E et F, sont délimités entre eux par une paroi commune 7 formée par la paroi de cloisonnement 7.1 du canal A à F situé à gauche, par une deuxième électrode 70 et par la paroi de cloisonnement 7.2 du canal A à F situé à droite. Chaque paroi commune 7 comprend ainsi une première paroi de cloisonnement 7.1, une deuxième électrode 70 et une deuxième paroi de cloisonnement 7.2 accolées. De l'extérieur vers l'intérieur, chaque paroi commune 7 comprend deux premières électrodes 73.1 et 73.2, deux éléments photocatalytiques 72.1 et 72.2, deux plaques diélectriques 71.1 et 71.2 et une seule électrode 70 qui est commune aux parois de cloisonnement 7.1 et 7.2. La deuxième électrode 70 est ainsi intercalée entre la plaque diélectrique 71.1 de la première paroi de cloisonnement 7.1 et entre la plaque diélectrique 71.2 de la deuxième paroi de cloisonnement 7.2.

La première paroi de cloisonnement 7.1 du canal A situé à gauche sur les figures 1 à 5, ainsi que la deuxième paroi de cloisonnement 7.2 du canal F situé à droite sur les figures 1 à 5, sont formées chacune par une première électrode 73.1 ou 73.2, un élément photocatalytique 72.1 ou 72.2, une plaque diélectrique 71.1 ou 71.2 et une deuxième électrode 70.

Les dimensions de chaque deuxième électrode 70 sont strictement inférieures à celles des plaques diélectriques 71.1 et 71.2, de sorte que les bords de chaque deuxième électrode 70 sont en retrait vers l'intérieur du canal A à F, c'est-à-dire vers une zone centrale de la paroi de cloisonnement 7.1 ou 7.2, par rapport aux bords des plaques diélectriques 71.1 et 71.2.

Les bords longitudinaux inférieurs de chaque paroi commune 7 sont reçus chacun séparément dans un support 46.1 (figure 3) qui est en appui contre la paroi inférieure 21.1 du caisson 2. Les bords longitudinaux supérieurs de chaque paroi commune 7 sont reçus chacun dans un support 46.2 qui est en appui contre la paroi supérieure 21.2 du caisson 2. Chaque support 46.1 et 46.2 est formé, d'une part, par un profilé 461.1 ou 461.2 à section globalement rectangulaire qui vient en contact avec la première électrode 73.1 ou 73.2 correspondante et, d'autre part, par une cornière 462.1 ou 462.2. Les profilés 461.1 et 461.2 comportent un gradin 461.3 qui reçoit la première électrode 73.1 ou 73.2 correspondante. La cornière 462.1 comprend une première aile qui est intercalée entre le bord longitudinal supérieur ou inférieur de la paroi de cloisonnement 7.1 ou 7.2, et une deuxième aile qui vient en contact avec la première électrode 73.1 correspondante. Plus précisément, la première électrode 73.1 ou 73.2 est reçue dans un gradin 462.3 de la deuxième aile de la cornière. Ainsi, chaque première électrode 73.1 et 73.2 est maintenue par deux supports 46.1 et 46.2 opposés, avec un jeu fonctionnel faible, voire nul.

De la même manière, les bords longitudinaux inférieurs de la paroi de cloisonnement 7.1 du canal A et les bords longitudinaux inférieurs de la paroi de cloisonnement 7.2 du canal F sont reçus dans un support 46'.1 et les bords longitudinaux supérieurs de la paroi de cloisonnement 7.1 du canal A et les bords longitudinaux supérieurs de la paroi de cloisonnement 7.2 du canal F sont reçus dans un support 46'.2.

Les supports 46'.1 et 46'.2 des canaux A et F sont fixés aux parois du caisson 2, par exemple au moyen de vis.

Six lampes 4 aptes à émettre un rayonnement photonique sont disposées dans chaque canal A à F (figure 5) et sont réparties le long de chaque canal A à F.

Le dispositif 1 comprend ainsi trente-six lampes 4. Les lampes 4 comprennent chacune un tube fluorescent en forme de U, qui comporte deux branches 4.1 et 4.2 parallèles au plan P et perpendiculaires à l'axe X, autrement dit parallèles à la direction de la plus grande dimension de la section transversale des canaux A à F. Chaque branche 4.1 et 4.2 est à égale distance des parois de cloisonnement 7.1 et 7.2 qui délimitent le canal A à F dans lequel est disposée la lampe 4 correspondante.

Un rayonnement photonique est un rayonnement lumineux, dans le domaine du visible ou de l'invisible. Le rayonnement photonique englobe les rayonnements ultraviolets, les rayonnements visibles et les rayonnements infrarouges. Ainsi, la longueur d'onde d'un rayonnement photonique est environ comprise entre 100 nm à 100 µm.

Les lampes 4 sont par exemple des lampes aptes à émettre un rayonnement ultraviolet. Par rayonnement ultraviolet, on entend un rayonnement dont la longueur d'onde est comprise dans le domaine des ultraviolets, c'est-à-dire entre 100 nm et 400 nm.

En variante, les lampes 4 sont aptes à émettre un rayonnement dans le domaine du visible, c'est-à-dire avec une longueur d'onde environ comprise entre 400 nm et 800 nm. Cette solution est plus facile à mettre en oeuvre car elle est compatible avec des lampes traditionnelles.

Une entretoise inférieure longitudinale 44.1 est disposée dans chaque canal A à F, contre la paroi inférieure 21.1 du caisson 2 et entre deux supports 46.1 ou un support 46.1 et un support 46'.1 des parois de cloisonnement 7.1 et 7.2 qui délimitent ce canal. Les entretoises inférieures 44.1 comportent chacune six encoches oblongues dans lesquelles sont positionnées les extrémités inférieures des tubes fluorescents 4.1 et 4.2 de chaque lampe 4.

Une entretoise supérieure longitudinale 44.2 (figure 4) est disposée dans chaque canal A à F, contre la paroi supérieure 21.2 du caisson 2 et entre deux supports 46.2 ou entre un support 46.2 et un support 46'.2 des parois de cloisonnement 7.1 et 7.2 délimitant ce canal.

L'extrémité supérieure 42.2 des tubes fluorescents 4.1 et 4.2 de chaque lampe 4 est maintenue par un support 41 commun qui dépasse à l'extérieur du caisson 2. Les entretoises supérieures 44.2 comportent chacune six ouvertures dans lesquelles sont disposés les supports 41 des lampes 4.

Les supports 41 s'étendent au travers de lumières 24 ménagées dans la paroi supérieure 21.2 du caisson 2. Des moyens de fixation 43, constitués par une patte et deux vis non représentées, sont utilisés pour assembler chaque lampe 4 à la paroi supérieure 21.2 du caisson 2.

Les supports inférieurs 46.1 et 46'.1 sont en contact avec les entretoises inférieures 44.1, de même que les supports supérieurs 46.2 et 46'.2 sont en contact avec les entretoises supérieures 44.2. Ainsi, les supports 46.1, 46'.1, 46.2 et 46'.2 et les entretoises 44.1 et 44.2 maintiennent les lampes 4 en position de manière précise. Les entretoises 44.1 et 44.2 définissent la largeur des canaux A à F.

Des plaques de support 23.1 et 23.2 extérieures sont montées sur les parois latérales 21.3 et 21.4 du caisson 2 par l'intermédiaire de plots 23.3 (figures 2 et 4), de sorte qu'un espace vide s'étend entre les plaques de support 23.1 et 23.3 et les parois latérales 21.3 et 21.4 du caisson 2.

Neuf ballasts 3.1 ou 3.2 sont montés sur chaque plaque de support 23.1 et 23.2, à l'opposé du caisson 2. Le générateur électrique 5.1 est relié électriquement à chaque ballast 3.1 et 3.2 par des câbles électriques C1.1 (figure 3). Comme représenté à la figure 5 uniquement pour le ballast 3.2 situé en bas à droite, chaque ballast 3.1 et 3.2 est relié électriquement à deux lampes 4 par des câbles électriques C1.2 qui cheminent à l'extérieur du caisson 2.

Le générateur électrique 5.2 est relié électriquement aux deux premières électrodes 73.1 et 73.2 et à la deuxième électrode 70 de chaque paroi de cloisonnement 7.1 et 7.2 par des câbles électriques C2.1 et C2.2. De préférence, pour assurer la sécurité, les câbles C2.1 et C2.2 sont adaptés à la haute tension.

La paroi inférieure 21.1 du caisson 2 comporte des trous 210.1 (figure 5) prévus chacun pour le passage de l'un des câbles électriques C2.1 alimentant les deuxièmes électrodes 73.1 et 73.2 qui font partie des parois de cloisonnement 7.1 et 7.2 délimitant chaque canal A à F. Les câbles électriques C2.1 sont connectés à des moyens de connexion de chaque première électrode 73.1 et 73.2, formés par exemple par des cosses étamées qui sont disposés le long du bord inférieur des deuxièmes électrodes 73.1 et 73.2.

Comme visible à la figure 2, la paroi supérieure 21.2 du caisson 2 comporte, pour chaque canal A à F, une encoche 210.2 qui est ménagée au niveau de l'extrémité longitudinale du caisson 2 située au premier plan à la figure 1. Cette encoche 210.2 est prévue pour le passage de l'un des câbles électriques C2.2. Les câbles électriques 2.2 sont reliés à des moyens de connexion de la deuxième électrode 70, non visibles sur les figures et formés par exemple par une languette métallique qui fait saillie vers l'extérieur du caisson 2 à partir du bord supérieur de la deuxième électrode 70.

Ainsi, l'alimentation des deuxièmes électrodes 70 s'effectue par leur bord supérieur, tandis que l'alimentation des premières électrodes 73.1 et 73.2 s'effectue par leur bord inférieur, à l'opposé du bord supérieur des premières électrodes 70. De cette manière, les risques d'amorçage d'arcs électrique sont réduits, voire éliminés. De plus, l'espace libre entre les câbles C2.1 et C2.2 et les bords des trous 210.1 et des encoches 210.2 sont remplis avec un mastic diélectrique, ce qui contribue également à éviter les risques d'amorçage d'arcs électriques.

En fonctionnement, les moyens de mise en dépression 8 font circuler le gaz à traiter entre l'entrée e et la sortie s de chaque canal A à F. Le générateur 5.1 alimente les lampes 4, qui génèrent un rayonnement photonique PH irradiant la face avant 722.1 et 722.2 des éléments photocatalytiques 72.1 et 72.2, ce qui crée une réaction de photocatalyse.

Simultanément, le générateur 5.2 crée une différence de potentiel électrique entre l'électrode 70 et les deux électrodes 73.1 et 73.2 des deux parois de cloisonnement 7.1 et 7.2 qui délimitent chaque canal A à F. Un plasma froid de surface 11 (figure 4) se forme ainsi contre les faces avant 712.1 et 712.2 des éléments photocatalytiques 72.1 ou 72.2 et contre les électrodes 73.1 et 73.2. Le plasma de surface 11 crée des vents ioniques favorisant l'homogénéisation du gaz traité, ce qui augmente l'efficacité de la photocatalyse.

La photocatalyse est induite simultanément par l'effet du rayonnement photonique PH et par le plasma de surface 11 sur les éléments photocatalytique 72.1 et 72.2, auquel vient s'ajouter la dégradation induite par le plasma de surface 11 lui-même. Cette combinaison améliore la rapidité globale de la réaction chimique de destruction des composés organiques présents dans le gaz.

La présence de plusieurs canaux A à F augmente le rendement du dispositif 1.

Les faces avant 722.1 et 722.2 des éléments photocatalytiques 72.1 et 72.2 sont irradiées avec une puissance homogène grâce à la présence de plusieurs lampes 4 placées au centre de chaque canal A à F. Ainsi, les effets du rayonnement photonique PH sont améliorés.

Le câblage des lampes 4 et des ballasts 3.1 et 3.2 est prévu pour allumer les rangées de lampes 4 séparément, ce qui permet notamment d'adapter le nombre de lampes 4 allumées en fonction de la concentration des éléments polluants présents dans le gaz à traiter. Ainsi, lorsque la concentration est faible, il est possible de ne pas allumer toutes les lampes 4, ce qui réduit la consommation électrique du dispositif 1

En variante non représentée, le caisson 2 n'est pas à section carrée. Par exemple, le caisson 2 peut être un tube creux à section rectangulaire.

Dans le cadre de l'invention, le nombre de canaux A à F et le nombre de lampes 4 peut varier.

Le montage du dispositif de traitement de gaz 1 est simple : l'opérateur assemble ensemble les parois 21.1, 21.2 et 21.3, de manière à former un ensemble présentant une section transversale en U. Puis, l'opérateur dispose successivement les éléments de chaque canal A à F contre le fond de cet ensemble, c'est-à-dire contre la paroi latérale 21.3, à savoir les parois de cloisonnement 7.1 et 7.2, les supports 46'.1, 46'.2, 46.1 et 46.2 et les entretoises 44.1 et 44.2. Puis, l'opérateur assemble la deuxième paroi latérale 21.4 du caisson 2 aux parois 21.1 et 21.2, de manière à ce que la section transversale du caisson 2 forme un contour fermé. Puis, il insère les lampes 4 dans les lumières 24 de la paroi supérieure 21.2 du caisson 2 et fixe les supports 41 des lampes 4 au caisson 2 au moyen des pattes 43 et de vis. Ainsi, les lampes 4 sont fixées en place dans les canaux A à F.

Les éléments photocatalytiques 72.1 et 72.2 sont amovibles. Lorsqu'il est nécessaire de les remplacer, il suffit de démonter le dispositif 1 et d'enlever les éléments photocatalytiques 72.1 et 72.2 usagés pour les remplacer avec des nouveaux éléments photocatalytiques.

Dans le cadre de l'invention, les modes de réalisation et variantes décrits peuvent être combinés entre eux, au moins partiellement.

## Revendications

1. Dispositif (1) de traitement de gaz, **caractérisé en ce qu'**il comprend un caisson (2) divisé en plusieurs canaux (A, B, C, D, E, F) comportant chacun une entrée (e) et une sortie (s) de gaz, au moins une lampe (4) générant un rayonnement photonique (PH), notamment ultraviolet, étant placée dans chaque canal (A-F) et chaque canal (A-F) étant délimité par deux parois de cloisonnement (7.1, 7.2) comprenant chacune :
- une paroi diélectrique (71.1, 71.2),
- un élément photocatalytique (72.1, 72.2) disposé contre la paroi diélectrique (71.1, 71.2) du côté du canal (A-F) et comprenant un support qui supporte un photocatalyseur,
- une première électrode (73.1, 73.2) disposée contre l'élément photocatalytique (72.1, 72.2), et
- une deuxième électrode (70) disposée contre la paroi diélectrique (71.1, 71.2).

2. Dispositif (1) selon la revendication 1, **caractérisé en ce que** chaque canal (A-F) présente une section transversale allongée et **en ce que**, le long de chaque canal (A-F), sont disposées plusieurs lampes (4) comprenant chacune un tube fluorescent (42.1, 42.2) qui s'étend notamment parallèlement à la plus grande dimension de la section transversale du canal (A-F).

3. Dispositif (1) selon l'une des revendications précédentes, **caractérisé en ce que** chaque élément photocatalytique (72.1, 72.2) se présente sous la forme d'une feuille et/ou **en ce que** chaque première électrode (73.1, 73.2) se présente sous la forme d'une grille.

4. Dispositif (1) selon l'une des revendications précédentes, **caractérisé en ce que** chaque seconde électrode (70) se présente sous la forme d'une plaque.

5. Dispositif (1) selon l'une des revendications précédentes, **caractérisé en ce que** chaque électrode (70, 73.1, 73.2) comprend des moyens de raccordement électrique, **en ce que** les moyens de raccordement électrique de chaque première électrode (73.1, 73.2) sont situés au niveau d'un premier de ses bords, et **en ce que** les moyens de raccordement électrique de chaque deuxième électrode (70) sont situés au niveau d'un second de ses bords, opposé audit premier bord des premières électrodes (73.1, 73.2).

6. Dispositif (1) selon l'une des revendications précédentes, **caractérisé en ce que** les bords de chaque deuxième électrode (70) sont en retrait vers l'intérieur du canal (A-F) par rapport aux bords des parois diélectriques (71.1, 71.2).

7. Méthode d'assemblage d'un dispositif (1) selon l'une des revendications précédentes, **caractérisée en ce qu'**elle comprend des étapes dans lesquelles :
- des parois (21.1, 21.2, 21.3) du caisson (2) sont assemblées entre elles due manière à former un ensemble présentant une section transversale en U,
- les éléments (70, 71.1, 72.1, 73.1, 73.2, 72.2, 71.2) délimitant chaque canal (A-F), dont les premières électrodes (73.1, 73.2) est les deuxièmes électrodes (70) sont disposés successivement contre le fond de cet ensemble, des entretoises (44.1, 44.2, 46.1, 46'.1, 46.2, 46'.2) définissant la largeur de chaque canal (A-F) étant intercalées entre les parois de cloisonnement (7.1,7.2),
- une paroi supplémentaire (21.4) du caisson (2) est assemblée de manière à ce que la section transversale du caisson (2) présente un contour fermé,
- les lampes (4) sont insérées dans des lumières (24) ménagées dans l'une des parois (21.1-21.4) du caisson (2) et fixées en place dans les canaux (A-F).

8. Système de traitement de gaz, **caractérisé en ce qu'**il comprend :
- un dispositif (1) selon l'une des revendications 1 à 6,
- un premier générateur électrique alternatif (5.1) alimentant les lampes (4),
- un deuxième générateur électrique alternatif (5.2) alimentant les électrodes (70, 73.1, 73.2),
- un organe de mise en dépression (8) faisant circuler le gaz à traiter entre l'entrée (e) et la sortie (s) de chaque canal (A-F).

9. Procédé de traitement de gaz au moyen d'un système selon la revendication 8, **caractérisé en ce qu'**il comprend des étapes dans lesquelles :
- l'organe de mise en dépression (8) fait circuler le gaz entre l'entrée (e) et la sortie (s) de chaque canal (A-F),
- les électrodes (70, 73.1, 73.2) génèrent un plasma froid de surface (11) contre les éléments photocatalytiques (72.1, 72.2),
- les lampes (4) génèrent un rayonnement photonique (PH) vers les éléments photocatalytiques (72.1, 72.2).

10. Procédé selon la revendication 9, **caractérisé en ce que** les étapes sont simultanées.

## Patentansprüche

1. Vorrichtung (1) zur Gasaufbereitung, **dadurch gekennzeichnet, dass** die Vorrichtung (1) ein Gehäuse (2) umfasst, das in mehrere Kanäle (A, B, C, D, E, F), die jeweils einen Gaseinlass (e) und einen Gasauslass (s) aufweisen, unterteilt ist, wobei mindestens eine Lampe (4), die eine insbesondere ultraviolette Photonenstrahlung (PH) erzeugt, in jedem Kanal (A-F) angeordnet ist und jeder Kanal (A-F) durch zwei Trennwände (7.1, 7.2) begrenzt ist, die jeweils Folgendes umfassen:
- eine dielektrische Wand (71.1, 71.2),
- ein photokatalytisches Element (72.1, 72.2), das auf der zum Kanal (A-F) weisenden Seite an der dielektrischen Wand (71.1, 71.2) angeordnet ist und einen Halter aufweist, der einen Photokatalysator hält,
- eine erste Elektrode (73.1, 73.2), die an dem photokatalytischen Element (72.1, 72.2) angeordnet ist, und
- eine zweite Elektrode (70), die an der dielektrischen Wand (71.1, 71.2) angeordnet ist.

2. Vorrichtung (1) nach Anspruch 1, **dadurch gekennzeichnet, dass** jeder Kanal (A-F) einen länglichen Querschnitt aufweist und dass entlang jedes Kanals (A-F) mehrere Lampen (4) angeordnet sind, die jeweils eine Leuchtröhre (42.1, 42.2) umfassen, welche sich insbesondere parallel zur größten Abmessung des Querschnitts des Kanals (A-F) erstreckt.

3. Vorrichtung (1) nach einem der vorhergehenden Ansprüche, **dadurch gekennzeichnet, dass** die photokatalytischen Elemente (72.1, 72.2) jeweils in Form eines Films ausgebildet sind und/oder dass die ersten Elektroden (73.1, 73.2) jeweils in Form eines Gitters ausgebildet sind.

4. Vorrichtung (1) nach einem der vorhergehenden Ansprüche, **dadurch gekennzeichnet, dass** die zweiten Elektroden (70) jeweils in Form einer Platte ausgebildet sind.

5. Vorrichtung (1) nach einem der vorhergehenden Ansprüche, **dadurch gekennzeichnet, dass** die Elektroden (70, 73.1, 73.2) jeweils elektrische Verbindungsmittel aufweisen, dass die elektrischen Verbindungsmittel der ersten Elektroden (73.1, 73.2) jeweils an einer ersten Kante derselben angeordnet sind und dass die elektrischen Verbindungsmittel der zweiten Elektroden (70) jeweils an einer der ersten Kante der ersten Elektroden (73.1, 73.2) gegenüberliegenden zweiten Kante derselben angeordnet sind.

6. Vorrichtung (1) nach einem der vorhergehenden Ansprüche, **dadurch gekennzeichnet, dass** die Kanten der zweiten Elektroden (70) gegenüber den Kanten der dielektrischen Wände (71.1, 71.2) jeweils in Richtung des Kanalinneren (A-F) zurückgesetzt sind.

7. Methode zum Zusammenbau einer Vorrichtung (1) nach einem der vorhergehenden Ansprüche, **dadurch gekennzeichnet, dass** die Methode Schritte umfasst, bei denen:
- Wände (21.1, 21.2, 21.3) des Gehäuses (2) so zusammengebaut werden, dass sie eine Anordnung mit einem U-förmigen Querschnitt bilden,
- die ersten Elektroden (73.1, 73.2) und die zweiten Elektroden (70) der jeweils die Kanäle (A-F) begrenzenden Elemente (70, 71.1, 72.1, 73.1, 73.2, 72.2, 71.2) nacheinander am Boden der Anordnung angeordnet werden, wobei die Größe des jeweiligen Kanals (A-F) definierende Abstandhalter (44.1, 44.2, 46.1, 46'.1, 46.2, 46'.2) zwischen die Trennwände (7.1, 7.2) eingefügt werden,
- eine zusätzliche Wand (21.4) des Gehäuses (2) so angebracht wird, dass der Querschnitt des Gehäuses (2) einen geschlossenen Umriss aufweist,
die Lampen (4) in Öffnungen (24), die in einer der Wände (21.1-21.4) des Gehäuses (2) aufgenommen sind, eingesetzt und in den Kanälen (A-F) befestigt werden.

8. System zur Gasaufbereitung, **dadurch gekennzeichnet, dass** das System Folgendes umfasst:
- eine Vorrichtung (1) nach einem der Ansprüche 1 bis 6,
- einen ersten Wechselstromgenerator (5.1), der die Lampen (4) versorgt,
- einen zweiten Wechselstromgenerator (5.2), der die Elektroden (70, 73.1, 73.2) versorgt,
- einen Unterdruckerzeuger (8), der das aufzubereitende Gas zwischen dem Gaseinlass (e) und dem Gasauslass (s) des jeweiligen Kanals (A-F) umwälzt.

9. Gasaufbereitungsverfahren mittels eines Systems nach Anspruch 8, **dadurch gekennzeichnet, dass** das Verfahren Schritte umfasst, bei denen:
- der Unterdruckerzeuger (8) das Gas zwischen dem Gaseinlass (e) und dem Gasauslass (s) des jeweiligen Kanals (A-F) umwälzt,
- die Elektroden (70, 73.1, 73.2) ein kaltes Oberflächenplasma (11) an den photokatalytischen Elementen (72.1, 72.2) erzeugen,
- die Lampen (4) eine Photonenstrahlung (PH) in Richtung der photokatalytischen Elemente (72.1, 72.2) erzeugen.

10. Verfahren nach Anspruch 9, **dadurch gekennzeichnet, dass** die Schritte gleichzeitig erfolgen.

## Claims

1. Gas treatment device (1), **characterised in that** it comprises a tank (2) divided into several channels (A, B, C, D, E, F), each having a gas inlet (e) and a gas outlet (s), wherein at least one lamp (4) that generates a photonic, in particular ultraviolet, radiation (PH) is placed in each channel (A-F) and each channel is delimited by two partition walls (7.1, 7.2), each comprising:
• a dielectric wall (71.1, 71.2),
• a photocatalytic element (72.1, 72.2) arranged between the dielectric wall (71.1, 71.2) on the side of the channel (A-F) and comprising a support, which carries a photocatalyst,
• a first electrode (73.1, 73.2) laid against the photocatalytic element (72.1, 72.2) and
• a second electrode (70) laid against the dielectric wall (71.1, 71.2).

2. Device (1) according to claim 1, **characterised in that** each channel (A-F) has an elongated cross-section and **in that** several lamps (4) are arranged along each channel (A-F), each of which lamps comprising a fluorescent tube (42.1, 42.2), which in particular extends parallel to the largest dimension of the cross-section of the channel (A-F).

3. Device (1) according to one of the preceding claims, **characterised in that** each photocatalytic element (72.1, 72.2) is provided in the form of a sheet and/or **in that** each first electrode (73.1, 73.2) is provided in the form of a grid.

4. Device (1) according to one of the preceding claims, **characterised in that** each second electrode (70) is provided in the form of a plate.

5. Device (1) according to one of the preceding claims, **characterised in that** each electrode (70, 73.1, 73.2) comprises electrical connection means, **in that** the electrical connection means of each first electrode (73.1, 73.2) are located at the level of a first of its edges, and **in that** the electrical connection means of each second electrode (70) are located at the level of a second of its edges opposed to said first edge of the first electrodes (73.1, 73.2).

6. Device (1) according to one of the preceding claims, **characterised in that** the edges of each second electrode (70) are set back towards the interior of the channel (A-F) in relation to the edges of the dielectric walls (71.1, 71.2).

7. Method of assembling a device (1) according to one of the preceding claims, **characterised in that** it comprises steps, in which:
• walls (21.1, 21.2, 21.3) of the tank (2) are assembled together to form a body having a U-shaped cross-section,
• the elements (70, 71.1, 72.1, 73.1, 73.2, 72.2, 71.2) delimiting each channel (A-F), of which the first electrodes (73.1, 73.2) and the second electrodes (70) are arranged successively against the base of this body, with cross bars (44.1, 44.2, 46.1, 46'.1, 46.2, 46'.2) that define the width of each channel (A-F) being interposed between the partition walls (7.1, 7.2),
• an additional wall (21.4) of the tank (2) is assembled so that the cross-section of the tank (2) has a closed contour,
• the lamps (4) are inserted into openings (24) arranged in one of the walls (21.1-21.4) of the tank (2) and fixed in place in the channels (A-F).

8. Gas treatment system, **characterised in that** it comprises:
• a device (1) according to one of claims 1 to 6,
• a first alternating current electric generator (5.1) supplying the lamps (4),
• a second alternating current electric generator (5.2) supplying the electrodes (70, 73.1, 73.2),
• a vacuum element (8) that causes the gas to be treated to circulate between the inlet (e) and the outlet (s) of each channel (A-F).

9. Gas treatment process using a system according to claim 8, **characterised in that** it comprises steps, in which:
• the vacuum element (8) causes the gas to circulate between the inlet (e) and the outlet (s) of each channel (A-F),
• the electrodes (70, 73.1, 73.2) generate a cold surface plasma (11) against the photocatalytic elements (72.1, 72.2),
• the lamps (4) generate a photonic radiation (PH) towards the photocatalytic elements (72.1, 72.2).

10. Process according to claim 9, **characterised in that** the steps are simultaneous.
